(19) Europäisches Patentamt
European Patent Office
Office européen des brevets

(11) **EP 2 236 504 A1**

(12) **EUROPEAN PATENT APPLICATION**
published in accordance with Art. 153(4) EPC

(43) Date of publication:
**06.10.2010 Bulletin 2010/40**

(51) Int Cl.:
***C07D 307/79*** (2006.01)  ***C07B 61/00*** (2006.01)

(21) Application number: **09706492.7**

(22) Date of filing: **23.01.2009**

(86) International application number:
**PCT/JP2009/051523**

(87) International publication number:
**WO 2009/096499 (06.08.2009 Gazette 2009/32)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **28.01.2008 JP 2008015948**

(71) Applicant: **Sumitomo Chemical Company, Limited Tokyo 104-8260 (JP)**

(72) Inventors:
• **YAMAUCHI, Kazuhiro**
  **Ibaraki-shi**
  **Osaka - (JP)**
• **MIYAMOTO, Takashi**
  **Nishinomiya-shi**
  **Hyogo 663-8101 (JP)**

(74) Representative: **Winter, Brandl, Fürniss, Hübner, Röss, Kaiser, Polte - Partnerschaft**
  **Alois-Steinecker-Strasse 22**
  **85354 Freising (DE)**

(54) **PROCESS FOR PRODUCTION OF 4-VINYL-2,3-DIHYDROBENZOFURAN**

(57)     A process for production of 4-vinyl-2,3-dihydrobenzofuran, which comprises a step including a reaction of 4-halo-2,3-dihydrobenzofuran with ethylene in the presence of a palladium compound and a base to produce crude 4-vinyl-2,3-dihydrobenzofuran, and a step of supplying the crude 4-vinyl-2,3-dihydrobenzofuran continuously or intermittently to a distillation apparatus to distill 4-vinyl-2,3-dihydrobenzofuran.

EP 2 236 504 A1

**Description**

TECHNICAL FIELD

The present invention relates to a process for producing 4-vinyl-2,3-dihydrobenzofuran.

BACKGROUND ART

4-Vinyl-2,3-dihydrobenzofuran is a compound which is useful as a synthesis intermediate for medicaments and the like, and as a production process therefor, for example, a process including reacting 4-chloro-2,3-dihydrobenzofuran with a vinyltin compound in the presence of a palladium catalyst (for example, see Tetrahedron Lett., 41, 4011 (2000)) and the like were known. However, such processes had problems in view of yield, and could not be considered to be industrially satisfying processes.

Furthermore, as a process for obtaining 4-vinyl-2,3-dihydrobenzofuran from a reaction mixture, a process including distilling at 40°C/0.5 mmHg (67 Pa) (for example, see Org. Proc. Res. Dev., 7, 547 (2003)) was known. However, it was difficult to distill under such high vacuum condition in industry.

DISCLOSURE OF THE INVENTION

Under such circumstances, the present inventors have made intensive studies aiming at developing a process where 4-vinyl-2,3-dihydrobenzofuran is efficiently obtained on an industrial scale, and consequently have achieved the present invention.

Namely, the present invention provides the following inventions.

<1> A process for production of 4-vinyl-2,3-dihydrobenzofuran, which comprises a step including a reaction of 4-halo-2,3-dihydrobenzofuran with ethylene in the presence of a palladium compound and a base to produce crude 4-vinyl-2,3-dihydrobenzofuran, and a step of supplying the crude 4-vinyl-2,3-dihydrobenzofuran continuously or intermittently to a distillation apparatus to distill 4-vinyl-2,3-dihydrobenzofuran.

<2> The process for production according to <1>, wherein the relationship between the temperature T (K) of a heat source of the distillation apparatus and the average residence time t (minute) of 4-vinyl-2,3-dihydrobenzofuran satisfies the following formula (1):

$$\ln(t) \leqq (5749/T) - 9.16 \qquad (1).$$

<3> The process for production according to <1> or <2>, wherein the temperature T of the heat source of the distillation apparatus is 323 to 423 K, and the average residence time t of 4-vinyl-2,3-dihydrobenzofuran is 0.01 to 60 minutes.

<4> The process for production according to any one of <1> to <3>, wherein the 4-halo-2,3-dihydrobenzofuran is 4-bromo-2,3-dihydrobenzofuran.

<5> The process for production according to any one of <1> to <4>, wherein the palladium compound is a divalent palladium compound.

<6> The process for production according to <5>, wherein the divalent palladium compound is palladium(II) acetate or palladium(II) chloride.

<7> The process for production according to any one of <1> to <6>, wherein the reaction of 4-halo-2,3-dihydrobenzofuran with ethylene is carried out further in the presence of a phosphine compound.

<8> The process for production according to <7>, wherein the phosphine compound is tri-o-tolylphosphine.

<9> The process for production according to any one of <1> to <8>, wherein the base is a tertiary amine compound.

<10> The process for production according to <9>, wherein the tertiary amine compound is triethylamine.

<11> The process for production according to any one of <1> to <10>, wherein the reaction of 4-halo-2,3-dihydrobenzofuran with ethylene is carried out in the presence of a solvent.

<12> The process for production according to <11>, wherein the solvent is at least one solvent selected from a nitrile solvent, an amide solvent and an ether solvent, or a mixed solvent thereof with water.

<13> The process for production according to <11>, wherein the solvent is acetonitrile, N,N-dimethylformamide, or a mixed solvent of any of them and water.

<14> A process for purification of 4-vinyl-2,3-dihydrobenzofuran, which includes a step for distilling 4-vinyl-2,3-dihydrobenzofuran, wherein crude 4-vinyl-2,3-dihydrobenzofuran is supplied continuously or intermittently to a distillation apparatus in said step.

<15> The process for purification according to <14>, wherein the relationship between the temperature T (K) of a heat source of the distillation apparatus and the average residence time t (minute) of 4-vinyl-2,3-dihydrobenzofuran satisfies the following formula (1):

$$\ln(t) \leqq (5749/T) - 9.16 \qquad (1).$$

<16> The process for purification according to <14> or <15>, wherein the temperature T of the heat source of the distillation apparatus is 323 to 423 K, and the average residence time t of 4-vinyl-2,3-dihydrobenzofuran is 0.01 to 60 minutes.

EFFECT OF THE INVENTION

According to the present invention, 4-vinyl-2,3-dihydrobenzofuran which is useful as a synthesis intermediate for medicaments and the like can be efficiently obtained on an industrial scale, which is industrially advantageous.

BEST MODE FOR CARRYING OUT THE INVENTION

Hereinafter a production process of the present invention is described in detail. First, a step for obtaining crude 4-vinyl-2,3-dihydrobenzofuran is explained.

In the production process of the present invention, the step for obtaining crude 4-vinyl-2,3-dihydrobenzofuran includes the reaction of 4-halo-2,3-dihydrobenzofuran with ethylene in the presence of a palladium compound and a base (hereinafter this reaction is referred to as the present reaction).

The 4-halo-2,3-dihydrobenzofuran can be produced according to, for example, a known process described in Tetrahedron Lett., 41, 4011 (2000) or the like. Examples of the 4-halo-2,3-dihydrobenzofuran may include 4-chloro-2,3-dihydrobenzofuran, 4-bromo-2,3-dihydrobenzofuran and 4-iodo-2,3-dihydrobenzofuran. 4-Bromo-2,3-dihydrobenzofuran and 4-iodo-2,3-dihydrobenzofuran are preferable in view of reactivity, of which 4-bromo-2,3-dihydrobenzofuran is more preferable in view of economic efficiency.

With regard to ethylene, a commercially available ethylene gas is generally used, or ethylene generated by any known process may be used. The used amount of ethylene may be 1 time by mole or more with respect to that of the 4-halo-2,3-dihydrobenzofuran, and the upper limit thereof is not specifically defined.

Examples of the base include tertiary amine compounds such as trimethylamine, triethylamine, tributylamine and N-methylmorpholine; secondary amine compounds such as diethylamine; aromatic amine compounds such as pyridine; strongly-basic amine compounds such as 1,8-diazabicyclo [5.4.0]-7-undecene; carboxylates such as sodium acetate and potassium acetate; phosphates such as sodium phosphate; and carbonates such as sodium carbonate and potassium carbonate. In the present invention, tertiary amine compounds are preferable, and triethylamine is more preferable. The used amount of the base may be 1 time by mole or more with respect to that of the 4-halo-2, 3-dihydrobenzofuran and the upper limit thereof is not specifically defined, and the amount is generally 1 to 10 times by mole with respect to that of the 4-halo-2,3-dihydrobenzofuran since the economic efficiency tends to become disadvantageous when the amount is too much.

Examples of the palladium compound include divalent palladium compounds such as palladium(II) chloride, palladium(II) bromide, palladium(II) iodide, palladium(II) acetate, palladium(II) propionate, palladium(II) trifluoroacetate, palladium(II) sulfate, palladium(II) acetylacetonate, palladium(II) cyanide, dichlorobis(triphenylphosphine)palladium(II), dichlorobis(tri-o-tolylphosphine)palladium(II), 1,2-bis(diphenylphosphino)ethanedichloropalladium(II) and [1,1'-bis (diphenylphosphino)ferrocene]dichloropalladium(II), and zerovalent palladium compounds such as tetrakis (triphenylphosphine)palladium(0). In the present reaction, divalent palladium compounds are preferable, and palladium(II) acetate or palladium(II) chloride is more preferable in view of availability. The used amount of the palladium compound may be 0.001 mol% or more with respect to that of the 4-halo-2,3-dihydrobenzofuran and the upper limit thereof is not specifically defined, and the amount is generally 0.002 to 10 mol%, preferably 0.05 to 2 mol% with respect to that of the 4-halo-2,3-dihydrobenzofuran since the economic efficiency tends to become disadvantageous when the amount is too much.

The present reaction may be carried out further in the presence of a phosphine compound. Examples of the phosphine compound include triphenylphosphine, tri-o-tolylphosphine, tri-m-tolylphosphine, tri-p-tolylphosphine, dimethylphenylphosphine, diethylphenylphosphine, ethyldiphenylphosphine, methyldiphenylphosphine, tributylphosphine, triethylphosphine, tricyclohexylphosphine, 1,2-bis(diphenylphosphino)ethane, 1,3-bis(diphenylphosphino)propane, and 1,4-bis(diphenylphosphino)butane. In the present invention, tri-o-tolylphosphine is preferable. The used amount of the phosphine compound may be 1 time by mole or more with respect to that of the palladium compound and the upper limit thereof is not specifically defined, and the amount is generally 1 to 50 times by mole, preferably 2 to 10 times by mole with respect to that of the palladium compound since the economic efficiency tends to become disadvantageous when the amount is too much. A more preferable combination of the palladium compound and phosphine compound is a combination of palladium(II) acetate and tri-o-tolylphosphine.

The present reaction is generally carried out in the presence of a solvent. Examples of the solvent include nitrile solvents such as acetonitrile and propionitrile; amide solvents such as N,N-dimethylformamide, N,N-dimethylacetamide and N-methylpyrrolidone; ether solvents such as 1,2-dimethoxyethane and tetrahydrofuran; ketone solvents such as acetone and methyl ethyl ketone; alcohol solvents such as methanol, ethanol and isopropanol; ester solvents such as ethyl acetate and propyl acetate; aromatic hydrocarbon solvents such as toluene and xylene; aliphatic hydrocarbon solvents such as hexane and heptane; and water. These may be used solely or as a mixed solvent of two or more kinds thereof. The above-mentioned solvent is preferably at least one solvent selected from a nitrile solvent, an amide solvent and an ether solvent, or a mixed solvent thereof with water, more preferably acetonitrile or N,N-dimethylformamide, or a mixed solvent thereof with water. The amount used of the solvent is not specifically limited, and is generally 1 to 10 times by weight with respect to that of the 4-halo-2,3-dihydrobenzofuran.

The present reaction is carried out by mixing the palladium compound, base, 4-halo-2,3-dihydrobenzofuran and ethylene, in the presence of the solvent if necessary, and the mixing process therefor is not specifically limited. Generally, the reaction is carried out by mixing a mixture of the palladium compound, base and 4-halo-2,3-dihydrobenzofuran under an ethylene atmosphere. When a phosphine compound is used, the phosphine compound may be mixed together with the palladium compound, base and 4-halo-2,3-dihydrobenzofuran; or the palladium compound and phosphine compound may be mixed in advance and the mixture may be added to a mixture of the base and 4-halo-2,3-dihydrobenzofuran.

The reaction temperature is generally 0 to 100°C, preferably 30 to 80°C. When the reaction temperature is too low, the reaction velocity decreases, and when the reaction temperature is too high, a problem may be caused in view of the stability of the product.

The present reaction is carried out under an ethylene atmosphere as mentioned above, and the pressure therefor is generally an ordinary pressure or more. The pressure is preferably 0.2 MPa or more, more preferably 0.4 to 3 MPa.

The reaction time varies depending on reaction conditions such as kinds and amounts of the palladium compound and base, the pressure of ethylene and the reaction temperature, and it is generally 1 to 24 hours. Progression of the reaction can be confirmed by a general means such as gas chromatography and high performance liquid chromatography.

By the present reaction, a reaction mixture containing 4-vinyl-2,3-dihydrobenzofuran is obtained.

The reaction mixture obtained by the present reaction further contains a hydrogen halide salt of a base, it is generally subjected to the distillation step as described below after the salt is removed. Examples of the treatment for removing such a hydrogen halide salt of the base include filtration treatment and washing treatment and the like. The washing treatment is preferable. After the filtration treatment is carried out, the obtained filtrate may be subjected to the washing treatment. The washing treatment is generally carried out using water. When a solvent miscible with water is used as the above-mentioned reaction solvent, the organic layer may be separated by washing by further using an organic solvent immiscible with water such as toluene and ethyl acetate. Alternatively, when a mixed solvent containing water is used as the above-mentioned reaction solvent and the liquid phases of the reaction mixture are separated into two layers, the layers may be subjected to liquid separation treatment, the obtained layer containing higher amount of water may be subjected to extraction treatment using an organic solvent immiscible with water; the obtained organic layer, the layer containing lower amount of water obtained by the previous liquid separation treatment, and if necessary, an organic solvent immiscible with water may be combined; and the obtained mixture may be washed using water.

Since the reaction mixture after removal of the hydrogen halide salt of the base by the above-mentioned removal treatment is a mixture with the solvent, it is generally subjected to concentration treatment, and then subjected to the distillation step described below. In the present invention, the treated product of the reaction mixture refers to a mixture containing 4-vinyl-2,3-dihydrobenzofuran, which is obtained by such removing treatment or concentration treatment.

In the present specification, the crude 4-vinyl-2,3-dihydrobenzofuran may be a reaction mixture obtained from the present reaction, or may be the treated product of the reaction mixture.

Next, the distillation step which is carried out while supplying the crude 4-vinyl-2,3-dihydrobenzofuran continuously or intermittently to a distillation apparatus is described. In the present invention, the "supplying continuously" refers to an embodiment in which a predetermined amount of crude 4-vinyl-2,3-dihydrobenzofuran is continuously supplied to a distillation apparatus without cease, and the "supplying intermittently" refers to an embodiment in which an operation in which a part of the predetermined amount of crude 4-vinyl-2,3-dihydrobenzofuran is supplied to a distillation apparatus to distill 4-vinyl-2,3-dihydrobenzofuran is repeated plural times until supplying of the predetermined amount of crude 4-vinyl-2,3-dihydrobenzofuran to the distillation apparatus is completed.

Examples of the distillation apparatus include distillation apparatuses such as a batchwise distillation apparatus, a centrifugal molecular distillation apparatus and a thin-film distillation apparatus. The distillation operation may be carried out by adjusting a heat supplying portion (also referred to as a heat source of the distillation apparatus) of these distillation apparatuses to a predetermined temperature (generally distillation temperature) in advance, and supplying the reaction mixture or treated product thereof obtained by the above-mentioned reaction continuously or intermittently to the distillation apparatus. In the present invention, it is preferable to use a thin-film distillation apparatus in view of heating time and evaporation efficiency.

The distillation is preferably carried out so that the relationship between the temperature T (K) of the heat source of the distillation apparatus and the average residence time t (minute) of 4-vinyl-2,3-dihydrobenzofuran satisfies the following formula (1):

$$\ln(t) \leq (5749/T) - 9.16 \qquad (1).$$

The temperature T of the heat source of the distillation apparatus (hereinafter referred to as temperature T) is generally 323 to 423 K (50 to 150°C), preferably in the range of 353 to 413 K (80 to 140°C), and more preferably in the range of 373 to 403 K (100 to 130°C).

In the present invention, the average residence time t (hereinafter referred to as time t) refers to an average value of the time through which 4-vinyl-2,3-dihydrobenzofuran is exposed to the temperature T. When a distillation apparatus of a type in which a thin-film is formed in an apparatus such as a centrifugal molecular distillation apparatus and a thin-film distillation apparatus is used, the time t is a value obtained by dividing the weight of 4-vinyl-3,4-dihydrobenzofuran contained in the thin-film formed in the apparatus by the weight of the evaporated 4-vinyl-3,4-dihydrobenzofuran per a unit time. When crude 4-vinyl-2,3-dihydrobenzofuran is supplied continuously to the batchwise distillation apparatus, the time t is a value obtained by dividing the weight of 4-vinyl-3,4-dihydrobenzofuran contained in the liquid at the reboiler part of the column bottom by the weight of the evaporated 4-vinyl-3,4-dihydrobenzofuran per a unit time. When crude 4-vinyl-2,3-dihydrobenzofuran is supplied intermittently to the batchwise distillation apparatus, the value obtained by dividing the average value of the weight of 4-vinyl-2,3-dihydrobenzofuran contained in the liquid at the reboiler portion of the column bottom of the distillation apparatus by the average value of the weight of the evaporated 4-vinyl-2,3-dihydrobenzofuran per a unit time over the entire period may be used as the time t. The time t is generally in the range of 0.01 to 60 minutes, and the time t is preferably short when the temperature T is high. The distillation is more preferably carried out in such a manner that the temperature T becomes 323 to 423 K (50 to 150°C) and the time t becomes 0.01 to 60 minutes.

The pressure in the distillation apparatus depends on the temperature T, and may be a pressure at which 4-vinyl-2,3-dihydrobenzofuran is distilled under the condition of the temperature T. It is generally in the range of 0.1 to 3 kPa. It is preferable to adjust the pressure so that the time t satisfies the above-mentioned formula 1.

It is preferable to use a polymerization inhibitor for distillation. Examples of the polymerization inhibitor include phenol compounds such as hydroquinone, 2,5-di-t-butylhydroquinone and 4-t-butylcatechol; benzoquinone compounds such as p-benzoquinone, o-benzoquinone and 2,5-di-t-butyl-p-benzoquinone; and N-oxyradical compounds such as 2,2,6,6-tetramethylpiperidine-N-oxyl. Such a polymerization inhibitor may be used by mixing it with the crude 4-vinyl-2,3-dihydrobenzofuran in advance, or may be used after putting it to the distillation apparatus in advance. The used amount of the polymerization inhibitor is not specifically limited, and it is generally 0.001 to 1% by weight with respect to that of the crude 4-vinyl-2,3-dihydrobenzofuran.

A high boiling point solvent may also be used for distillation. Examples of the high boiling point solvent may be a solvent whose boiling point under the condition of the pressure in the distillation apparatus is higher than that of 4-vinyl-2,3-dihydrobenzofuran, and examples include liquid paraffin, and ethylene glycol. Such a high boiling point solvent may be used after mixing it with the crude 4-vinyl-2,3-dihydrobenzofuran in advance, or may be used after putting it into the distillation apparatus in advance. The used amount of the high boiling point solvent is not specifically limited, and it is generally 0.1 to 5 times by weight with respect to that of the crude 4-vinyl-2,3-dihydrobenzofuran.

A process for purification of 4-vinyl-2,3-dihydrobenzofuran, which includes a step for distilling 4-vinyl-2,3-dihydrobenzofuran, wherein crude 4-vinyl-2,3-dihydrobenzofuran is supplied continuously or intermittently to a distillation apparatus in said step is also one of the present invention.

In the purification process of the present invention, the origin of the crude 4-vinyl-2,3-dihydrobenzofuran is not specifically limited. Examples of the crude 4-vinyl-2,3-dihydrobenzofuran include a reaction mixture obtained by the reaction described in Tetrahedron Lett., 41, 4011 (2000) or Org. Proc. Res. Dev., 7, 547 (2003) or a treated product thereof, and a reaction mixture obtained by the present reaction or a treated product thereof. The reaction mixture obtained by the present reaction or a treated product thereof is preferable.

The distillation step in the purification process is the same as the distillation in the production process of the present invention.

The production process and purification process of the present invention each may further include a step for purifying 4-vinyl-2,3-dihydrobenzofuran by column chromatography, recrystallization or the like.

EXAMPLES

Hereinafter the present invention is further described in detail by examples, but the present invention should not be limited by these examples. Meanwhile, the "recovery ratio" after distillation in each example below was obtained by dividing the weight of 4-vinyl-2,3-dihydrobenzofuran contained in a distilled liquid by the weight of 4-vinyl-2,3-dihydrobenzofuran contained in crude 4-vinyl-2,3-dihydrobenzofuran subjected to distillation. Furthermore, the "retention ratio" was obtained by dividing the total weight of 4-vinyl-2,3-dihydrobenzofuran contained in a distilled liquid and distillation residue, respectively, by the weight of 4-vinyl-2,3-dihydrobenzofuran contained in crude 4-vinyl-2,3-dihydrobenzofuran subjected to distillation.

Example 1 (Preparation of reaction mixture containing 4-vinyl-2,3-dihydrobenzofuran)

Into a 200 mL-volume autoclave, 5.0 g (25 mmol) of 4-bromo-2,3-dihydrobenzofuran, 7.5 g of acetonitrile, 4.0 g of water and 5.08 g (50 mmol) of triethylamine were charged. The space in the reaction vessel was made under a nitrogen atmosphere, and thereafter 28 mg (0.13 mmol) of palladium acetate and 153 mg (0.5 mmol) of tri-o-tolylphosphine were added thereto. Ethylene was added to the reaction system to pressurize up to the internal pressure of 0.8 MPa at room temperature, and the internal temperature was raised to 70°C while the reaction mixture was stirred. After the temperature was raised, the internal pressure was adjusted to 0.9 MPa, and stirring was carried out at 70 to 75°C for 4 hours while the pressure was kept. After the reaction was completed, the solid content was filtered off, and thereafter the obtained filtrate was subjected to liquid separation treatment to give an organic layer. The aqueous layer was mixed with 15.0 g of toluene, and extraction treatment was carried out to give an organic layer. The above-mentioned two organic layers were combined and washed with 7.5 g of water to give 24.5 g of a brown solution. When the solution was analyzed by high performance liquid chromatography, 12.7% by weight of 4-vinyl-2,3-dihydrobenzofuran was contained in the solution. The yield was 85%.

Example 2 (Preparation of reaction mixture containing 4-vinyl-2,3-dihydrobenzofuran)

Into a 50 mL-volume autoclave, 1.0 g (5 mmol) of 4-bromo-2,3-dihydrobenzofuran, 1.5 g of acetonitrile, 0.8 g of water and 1.02 g (010 mmol) of triethylamine were charged. The gas phase portion in the reaction vessel was made a nitrogen atmosphere, and thereafter 5.6 mg (0.026 mmol) of palladium acetate and 30.6 mg (0.1 mmol) of tri-o-tolylphosphine were added. Ethylene was added to the reaction system to pressurize up to an internal pressure of 1.3 MPa at room temperature, and the internal temperature was raised to 80°C while stirring. After the temperature was raised, the internal pressure was adjusted to 1.4 MPa, and stirring was carried out at 75 to 80°C for 8 hours while the pressure was kept. After the reaction was completed, the obtained mixture was mixed with 3.0 g of toluene and 3.0 g of water and was subjected to liquid separation treatment, and the obtained organic layer was sequentially washed with 5% by weight of aqueous hydrochloric acid and 4% by weight of an aqueous sodium hydrogen carbonate solution to give 3. 53 g of a brown solution. When the solution was analyzed by high performance liquid chromatography, 18.1% by weight of 4-vinyl-2,3-dihydrobenzofuran was contained in the solution. The yield was 87%.

Example 3 (Preparation of reaction mixture containing 4-vinyl-2,3-dihydrobenzofuran)

The reaction and post-treatment were carried out in the same manner as in Example 2 except that N, N-dimethylformamide was used instead of acetonitrile in Example 2, to give 3.50 g of a brown solution. When the solution was analyzed by high performance liquid chromatography, 18.3% by weight of 4-vinyl-2,3-dihydrobenzofuran was contained in the solution. The yield was 87%.

Example 4 (Distillation in which crude 4-vinyl-2,3-dihydrobenzofuran is supplied continuously to batchwise distillation apparatus)

A reaction mixture obtained in the same manner as in Example 1 was concentrated at 40 to 50°C under a pressure reduction degree of 1.3 to 27 kPa to give 42.8 g of crude 4-vinyl-2,3-dihydrobenzofuran (the content of 4-vinyl-2,3-dihydrobenzofuran was 84.8% by weight).

Into a 50 ml-volume three-necked flask equipped with an addition funnel, a stirring blade and a distillation connecting tube, 6.0 g of liquid paraffin and 6 mg of hydroquinone were charged. Further, 42.8 g of the above-mentioned crude 4-vinyl-2,3-dihydrobenzofuran was charged into the addition funnel. The three-necked flask was heated in an oil bath at 120°C (temperature T = 393 K), and the pressure in the apparatus was reduced to 2 mmHg (0.27 kPa). The content in the flask was stirred, and 4-vinyl-2,3-dihydrobenzofuran was distilled while adding dropwise the whole amount of the crude 4-vinyl-2,3-dihydrobenzofuran dropwise over 50 minutes. After the dropwise addition was completed, stirring of the content in the flask was continued while keeping the temperature of the oil bath and the pressure reduction degree for about 10 minutes until the distillation of 4-vinyl-2,3-dihydrobenzofuran was completed, to give 34.7 g of a distilled liquid. The average residence time (time t) of 4-vinyl-2,3-dihydrobenzofuran in the three-necked flask was about 10 minutes.

When the distilled liquid was analyzed by high performance liquid chromatography, the content of 4-vinyl-2,3-dihydrobenzofuran in the distilled liquid was 97.2% by weight. The recovery ratio of 4-vinyl-2,3-dihydrobenzofuran was 93%.

When the content of the three-necked flask was analyzed by high performance liquid chromatography, 2.0 g of 4-vinyl-2,3-dihydrobenzofuran was contained in the content. The total amount of 4-vinyl-2,3-dihydrobenzofuran in the distilled liquid and three-necked flask was 35.7 g, and the retention ratio was 98%.

In such purification by distillation, the relationship between the time t and temperature T is as follows, which satisfies the above-mentioned formula 1.

```
ln (t): 2.30 (= ln 10)

(5749/T) - 9.16: 5.46 (= (5749/393) - 9.16)
```

Example 5 (distillation in which crude 4-vinyl-2,3-dihydrobenzofuran is supplied continuously to thin film distillation apparatus)

The reaction mixture obtained in the same manner as in Example 1 was concentrated to give 11.34 kg of crude 4-vinyl-2,3-dihydrobenzofuran (the content of 4-vinyl-2,3-dihydrobenzofuran was 90.4% by weight).

The whole amount of the obtained crude 4-vinyl-2,3-dihydrobenzofuran and 3.03 kg of liquid paraffin were mixed, and the whole amount of the obtained mixture was distilled while the mixture was supplied continuously to an external jacket-type thin film distillation apparatus made of glass ("Wiprene (registered trademark)" manufactured by Kobelco Eco-Solutions Co. , Ltd. , heat transmission area 0.034 $m^2$, jacket temperature (temperature T) 393 K, pressure 0. 6 mmHg (0.08 kPa)) at a velocity of 3. 9 g/min to give 10.24 kg of a distilled liquid. Since the thickness of the thin film formed on the heat transmitting portion during distillation was 1 mm or less, and the density of the crude 4-vinyl-2,3-dihydrobenzofuran was 1.1 $g/cm^3$, the average residence time (time t) of the liquid was calculated as 10 minutes or less.

When the distilled liquid was analyzed by high performance liquid chromatography, the content of 4-vinyl-2, 3-dihydrobenzofuran in the distilled liquid was 98.8% by weight. The recovery ratio of 4-vinyl-2, 3-dihydrobenzofuran was 98.6%.

When the distilled residue was analyzed by high performance liquid chromatography, 0.10 kg of 4-vinyl-2,3-dihydrobenzofuran was contained in the content. The total amount of 4-vinyl-2,3-dihydrobenzofuran in the distilled liquid and distillation residue was 10.21 kg, and the retention ratio was 99.6%.

In such purification by distillation, the relationship between the time t and temperature T is as follows, which satisfies the above-mentioned formula 1.

```
ln (t): 2.30 (= ln 10) or less

(5749/T) - 9.16: 5.46 (= (5749/393) - 9.16)
```

Comparative Example 1 (distillation in which crude 4-vinyl-2,3-dihydrobenzofuran is supplied all at once to batchwise distillation apparatus)

37.3 g of a mixture obtained by concentrating the reaction mixture obtained in the same manner as in Example 1 at 40 to 50°C and a pressure reduction degree of 1.3 to 27 kPa (the content of 4-vinyl-2,3-dihydrobenzofuran was 88.4% by weight), 5.0 g of liquid paraffin and 5 mg of hydroquinone were charged into a three-necked flask equipped with a stirring blade and a distillation connecting tube. The three-necked flask was heated in an oil bath at 120°C, the pressure in the apparatus was reduced to 8 mmHg (1.1 kPa) under stirring. The pressure reduction degree in the flask was then gradually raised, and the pressure was finally reduced to 2 mmHg (0.27 kPa) to distill 4-vinyl-2,3-dihydrobenzofuran, whereby 25.4 g of a distilled liquid was obtained. The time required to complete the distillation was about 360 minutes.

When the distilled liquid was analyzed by high performance liquid chromatography, the content of 4-vinyl-2,3-dihydrobenzofuran in the distilled liquid was 98.7% by weight. The recovery ratio of 4-vinyl-2,3-dihydrobenzofuran was 76%.

The content in the three-necked flask was in the form of tar. When the content was analyzed by high performance liquid chromatography, only 2.5 g of 4-vinyl-2,3-dihydrobenzofuran was contained in the content, and the remainder was considered to be polymer. The total amount of 4-vinyl-2,3-dihydrobenzofuran in the distilled liquid and three-necked flask was 27.6 g, and the retention ratio was 84%.

In such purification by distillation, the relationship between the time t and temperature T is as follows, which does not satisfy the above-mentioned formula 1.

$$\ln (t): 5.88 \ (= \ln 360)$$

$$(5749/T) - 9.16: 5.46 (= (5749/393) - 9.16)$$

INDUSIRIAL APPLICABILITY

4-Vinyl-2,3-dihydrobenzofuran obtained by the present invention is useful as a synthesis intermediate for medicaments such as melatonin agonists (for example, see Japanese National Phase PCT Laid-open No. 2002-516859), and such a compound can be obtained with high purity. Thus, the present invention is industrially applicable.

**Claims**

1. A process for production of 4-vinyl-2,3-dihydrobenzofuran, which comprises a step including a reaction of 4-halo-2,3-dihydrobenzofuran with ethylene in the presence of a palladium compound and a base to produce crude 4-vinyl-2,3-dihydrobenzofuran, and a step of supplying the crude 4-vinyl-2,3-dihydrobenzofuran continuously or intermittently to a distillation apparatus to distill 4-vinyl-2,3-dihydrobenzofuran.

2. The process for production according to claim 1, wherein the relationship between the temperature T (K) of a heat source of the distillation apparatus and the average residence time t (minute) of 4-vinyl-2,3-dihydrobenzofuran satisfies the following formula (1):

$$\ln(t) \leqq (5749/T) - 9.16 \qquad (1).$$

3. The process for production according to claim 1 or 2, wherein the temperature T of the heat source of the distillation apparatus is 323 to 423 K, and the average residence time t of 4-vinyl-2,3-dihydrobenzofuran is 0.01 to 60 minutes.

4. The process for production according to any one of claims 1 to 3, wherein the 4-halo-2,3-dihydrobenzofuran is 4-bromo-2,3-dihydrobenzofuran.

5. The process for production according to any one of claims 1 to 4, wherein the palladium compound is a divalent palladium compound.

6. The process for production according to claim 5, wherein the divalent palladium compound is palladium(II) acetate or palladium(II) chloride.

7. The process for production according to any one of claims 1 to 6, wherein the reaction of 4-halo-2,3-dihydrobenzofuran with ethylene is carried out further in the presence of a phosphine compound.

8. The process for production according to claim 7, wherein the phosphine compound is tri-o-tolylphosphine.

9. The process for production according to any one of claims 1 to 8, wherein the base is a tertiary amine compound.

**10.** The process for production according to claim 9, wherein the tertiary amine compound is triethylamine.

**11.** The process for production according to any one of claims 1 to 10, wherein the reaction of 4-halo-2,3-dihydrobenzofuran with ethylene is carried out in the presence of a solvent.

**12.** The process for production according to claim 11, wherein the solvent is at least one solvent selected from a nitrile solvent, an amide solvent and an ether solvent, or a mixed solvent thereof with water.

**13.** The process for production according to claim 11, wherein the solvent is acetonitrile, N,N-dimethylformamide, or a mixed solvent of any of them and water.

**14.** A process for purification of 4-vinyl-2,3-dihydrobenzofuran, which includes a step for distilling 4-vinyl-2,3-dihydrobenzofuran, wherein crude 4-vinyl-2,3-dihydrobenzofuran is supplied continuously or intermittently to a distillation apparatus in said step.

**15.** The process for purification according to claim 14, wherein the relationship between the temperature T (K) of a heat source of the distillation apparatus and the average residence time t (minute) of 4-vinyl-2,3-dihydrobenzofuran satisfies the following formula (1):

$$\ln(t) \leqq (5749/T) - 9.16 \qquad (1).$$

**16.** The process for purification according to claim 14 or 15, wherein the temperature T of the heat source of the distillation apparatus is 323 to 423 K, and the average residence time t of 4-vinyl-2,3-dihydrobenzofuran is 0.01 to 60 minutes.

## INTERNATIONAL SEARCH REPORT

| | International application No. |
|---|---|
| | PCT/JP2009/051523 |

A.   CLASSIFICATION OF SUBJECT MATTER
*C07D307/79*(2006.01)i, *C07B61/00*(2006.01)i

According to International Patent Classification (IPC) or to both national classification and IPC

B.   FIELDS SEARCHED

Minimum documentation searched (classification system followed by classification symbols)
C07D307/79, C07B61/00

Documentation searched other than minimum documentation to the extent that such documents are included in the fields searched
Jitsuyo Shinan Koho          1922-1996    Jitsuyo Shinan Toroku Koho   1996-2009
Kokai Jitsuyo Shinan Koho    1971-2009    Toroku Jitsuyo Shinan Koho   1994-2009

Electronic data base consulted during the international search (name of data base and, where practicable, search terms used)
CAplus(STN), CASREACT(STN), REGISTRY(STN)

C.   DOCUMENTS CONSIDERED TO BE RELEVANT

| Category* | Citation of document, with indication, where appropriate, of the relevant passages | Relevant to claim No. |
|---|---|---|
| Y | ZHU, Jingyang et al., Copper(I)-catalyzed intramolecular cyclization reaction of 2-(2'-chlorophenyl)ethanol to give 2,3-dihydrobenzofuran, Tetrahedron Letters, 2000, 41(21),4011-4014 | 1-13 |
| X | RAO, Meena et al., A Practical Pilot-Scale Synthesis of 4-Vinyl-2,3-dihydrobenzofuran Using Imidate Ester Chemistry and Phase-Transfer Catalysis, Organic Process Research & Development, 2003, 7(4), 547-550 | 14-16 |
| Y | | 1-13 |
| Y | RUSSELL, Michael G. N. et al., Asymmetric synthesis of the northern segment of ephedradine C. A novel dihydrobenzo[b]furan formation, Tetrahedron Letters, 1999, 40(49), 8667-8670 | 1-13 |

| ☒ | Further documents are listed in the continuation of Box C. | ☐ | See patent family annex. |

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 April, 2009 (14.04.09) | 21 April, 2009 (21.04.09) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

International application No.

PCT/JP2009/051523

C (Continuation). DOCUMENTS CONSIDERED TO BE RELEVANT

| Category* | Citation of document, with indication, where appropriate, of the relevant passages | Relevant to claim No. |
|---|---|---|
| Y | KUROSAWA, Wataru et al., Total synthesis of (-)-ephedradine A: an efficient construction of optically active dihydrobenzofuran-ring via C-H insertion reaction, Tetrahedron, 2004, 60(43), 9615-9628 | 1-13 |
| Y | WO 2006/027680 A1 (PFIZER INC.), 16 March, 2006 (16.03.06), Full text; particularly, preparations 5, 6 (Family: none) | 1-13 |
| Y | JP 2003-260365 A (DAICEL CHEM IND LTD.), 16 September, 2003 (16.09.03), Full text & JP 4071015 B2 | 1-13 |

Form PCT/ISA/210 (continuation of second sheet) (April 2007)

**REFERENCES CITED IN THE DESCRIPTION**

**Patent documents cited in the description**

- JP 2002516859 W

**Non-patent literature cited in the description**

- *Tetrahedron Lett.,* 2000, vol. 41, 4011

- *Org. Proc. Res. Dev.,* 2003, vol. 7, 547